# EUROPEAN PATENT APPLICATION

(11) **EP 4 205 728 A1**
(43) Date of publication of application: **05.07.2023**
(21) Application number: 21864742.8
(22) Date of filing: 06.09.2021
(51) Int. Cl.: A61K 9/16, A61K 31/58, A61K 31/445, A61K 31/485, A61K 38/17, A61K 38/08

(54) **SUSTAINED-RELEASE MICROPARTICLES FOR SUSTAINED RELEASE OF DRUG**

(30) Priority: 07.09.2020 KR 20200113924
(71) Applicant: Inventage Lab Inc., Gyeonggi-do 13403 (KR)
(72) Inventor: KIM, Ju Hee, Seongnam-si, Gyeonggi-do 13622 (KR); KIM, Donghoon, Seongnam-si, Gyeonggi-do 13530 (KR); KIM, Seyeon, Suwon-si Gyeonggi-do 16682 (KR)
(74) Representative: BCKIP Part mbB
(86) International application number: PCT/KR2021/012001
(87) International publication number: WO 2022/050783

(57) **Abstract**

The present disclosure provides sustained-release microparticles comprising a biodegradable polymer and a drug, wherein the biodegradable polymer and the drug are uniformly distributed throughout the particles, and the microparticles do not show an initial excessive release of the drug, and are composed of uniform-sized particles having a particle size distribution width of 35 microns or less analyzed by a particle size analyzer and a specific surface area of the microparticles of 0.75×10⁻¹ to 2.0×10⁻¹ m²/g, thereby exhibiting a sustained release pattern of the drug. The injection composition comprising the drug contained in such microparticles can control the release of the drug for a selected period during injection administration to release an effective drug concentration constantly, and when formulated as an injection product, can reduce foreign body sensation and pain to the subject to enable an injection formulation with high compliance to be provided.

## Description

### Technical Field

The present disclosure relates to microparticles for sustained release of drugs. More specifically, the present disclosure relates to sustained-release microparticles that contain a drug in microparticles comprising a biodegradable polymer so that there is no excessive release at the initial stage of administration when the drug is administered, and the effective amount of the drug can be continuously released for a certain period of time.

### Background Art

In general, microparticles are one of the formulations of parenterally administered drugs consisting of a drug and a carrier used to control its carrying and release. Microparticles encapsulate a drug in a biodegradable polymer carrier having a property of being slowly decomposed in vivo, and release the drug to the body according as the decomposition of the polymer proceeds.

Recently, as high molecular weight peptides, proteins, or others have been developed as new therapeutic drugs, various efforts have been made to encapsulate these drugs in a polymer carrier and continuously release them. Aliphatic polyester, which is currently developed and used as a polymer carrier for protein and peptide drugs, has already been recognized for its biocompatibility and approved by the US Food and Drug Administration (FDA), and has widely been used as a drug delivery carrier, surgical suture, or the like. Specific examples of the aliphatic polyester include poly-L-lactic acid, polyglycolic acid, poly-D-lactic acid-co-glycolic acid, poly-L-lactic acid-co-glycolic acid, poly-D,L-lactic acid-co-glycolic acid (hereinafter referred to as 'PLGA'), poly-caprolactone, polyvalerolactone, poly-hydroxybutyrate, polyhydroxyvalerate, etc.

Despite various attempts to continuously release it by encapsulating the drug in such a polymer carrier, in the case of the formulation in which the protein drug is encapsulated in the microspheres made of the above-described aliphatic polyester, a problem of the initial excessive release (initial burst effect) of the drug, a problem in which the drug's effect is not exerted during a certain period because the release rate of the drug is not constantly controlled for a certain period of time, and a problem such as incomplete release in which the encapsulated drug is not released 100% appear unexpectedly so that there is a difficulty in selling it as an actual injectable formulation.

For example, in the case of model protein drugs such as bovine serum albumin and lysozyme, it has been reported that the final release amount is around 50% after a large amount of drug is initially released [Crotts, G. and Park, TG, J. Control Release, 44, 123-134, 1997; Leonard, N.B., Michael, L.H., Lee, M.M. J. Pharm. Sci., 84, 707-712], when recombinant human growth hormone is encapsulated in microspheres using aliphatic polyester as a carrier, 30 to 50% of a protein drug is initially excessively released, and then about 40 to 60% of the amount is not released and remains within the microspheres.

Such a problem is that, if the particle size of the finally formed microspheres is highly variable regardless of whether or not the microspheres are prepared by any one method of a phase separation method, a spray-drying method, and an emulsion solvent evaporation method, the amount of drug released from microspheres of different sizes is inevitably not constant.

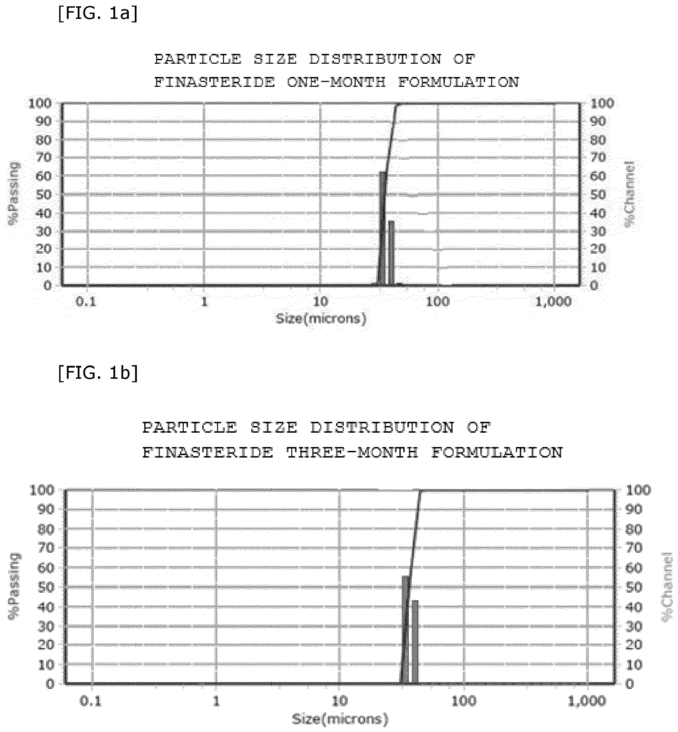

As an example, the microspheres prepared by the emulsion solvent evaporation method are disclosed in KR Registration Patent No. 10-2091114 that the particles of the prepared microspheres are polydisperse with a wide variety of sizes (see FIGS. 1A to 1I).

Therefore, in order to provide a drug at a sustained release rate for an accurate treatment period, the uniformity of particle size is of paramount importance. The particle size affects many properties of particulate matter and is an important indicator of particle quality and performance. The size and shape of the particles affect the fluidity and compressibility of the particles. Larger, more spherical particles will generally have the fluidity more easily than smaller or higher aspect ratio particles. Smaller particles will dissolve faster than larger particles and result in higher suspension viscosity [Sarlesh rajput, et al., A REVIEW ON MICROSPHERES: METHODS OF PREPARATION AND EVALUATION, World Journal of Pharmacy and Pharmaceutical Sciences, Volume 1, Issue 1, 422-438].

The particle size uniformity as well as the particle size may predict the release rate of drug more accurately than polydisperse microspheres with non-uniform particle size and ensure release sustainability for an accurate treatment period. Therefore, the uniform size of the particles is a problem that should be solved for sustained-release drugs.

Many microparticulate drugs developed so far still have a problem of initial excessive release (burst) and a problem such as incomplete control of the drug release rate during the treatment period, and one of the fundamental reasons for these problems will be non-uniformity of the particle size.

Therefore, the present applicant has studied intensively by focusing on completing sustained-release particles with guaranteed stability and reliability that can provide a drug in a controlled release pattern for a required period of time through the preparation of uniform particles.

### DISCLOSURE

### Technical Problem

Accordingly, it is an object of the present disclosure for the present applicant to provide microparticles for use in a sustained-release injection formulation of a drug, the microparticles which are prepared in an appropriate particle size that does not cause foreign body sensation and pain when administered as an injection product to a subject even if the biodegradable polymer and the drug are mixed at various ratios, and which are capable of exhibiting uniformity of drug release for a period necessary even for variations in the type and mixing ratio of biodegradable polymers and drugs without excessive drug release in the initial stage because sizes of the prepared particles have high uniformity.

### Technical Solution

In order to achieve the above object, the present disclosure provides sustained-release microparticles comprising a biodegradable polymer and a drug, wherein the microparticles are perfectly spherical, the biodegradable polymer and the drug are evenly distributed throughout the particles, and the sustained-release microparticles are composed of particles having a particle size distribution width of 35 microns or less analyzed by the particle size analyzer and a specific surface area per unit mass of 0.75×10⁻¹ to 2.0×10⁻¹ m²/g.

As an embodiment, the sustained-release microparticles do not exhibit an initial excessive release of the drug when analyzing the blood release pattern of the drug, but exhibit a sustained release pattern of the drug for a desired period for which drug administration is required.

In an embodiment, the biodegradable polymer is contained in an amount range of 60 to 97% by weight based on the weight of the microparticles.

In an embodiment, the sustained-release microparticles comprise the drug and the biodegradable polymer so that the drug and the biodegradable polymer are contained in a mixing ratio range of 1:1.5 to 1:30.

In an embodiment, the drug contained in the microparticles is selected from the group consisting of Donepezil, Aripiprazole, Olanzapine, Palonosetron, Minocycline, Memantine, Naltrexone, Alendronate, Deoxycholate, Risedronate, Ibandronate, Zoledronate, Liraglutide, Exenatide, Lanreotide, Octreotide, Deslorelin, Leuprorelin, Goserelin, Triptorelin, and Finasteride.

In an embodiment, the biodegradable polymer is one or more selected from the group consisting of poly-L-lactic acid, polylactide, polyglycolic acid, poly-D-lactic acid-co-glycolic acid, poly-L-lactic acid-co-glycolic acid, poly-D,L-lactic acid-co-glycolic acid, poly-caprolactone, polyvalerolactone, poly-hydroxybutyrate, and polyhydroxyvalerate.

In an embodiment, it is characterized in that the microparticles are perfectly sphere having an average diameter (D₅₀) between 20 um and 100 um.

In an embodiment, the sustained-release microparticles are characterized in that the ratio of the maximum blood concentration (Cₘₐₓ) to the initial blood concentration (Cᵢₙₜ) of the drug released from the microparticles is 2 to 30.

In an embodiment, it is characterized in that the microparticles exhibit sustained release of the drug for a period selected from 1 week to 12 months.

### Advantageous Effects

According to the microparticles of the present disclosure, the microparticles are composed of particles having a uniform particle size-showing narrow particle size distribution width and a specific surface area range, and are contained in a composition for injection to achieve control of the release rate of a drug upon injection into a patient, thereby providing the effect of enabling the drug to be continuously released for a desired period without causing the initial excessive release of the drug. Since it is possible to provide a drug with a stable release pattern for a desired selection period of time from such sustained-release microparticles, there are effects that the inconvenience that the drug should be taken every day is solved, and the problem of drug side effects or lack of efficacy due to initial excessive release or instability of release can be minimized.

### Brief Description of Drawings

FIG. 1a shows a particle size distribution graph of the microparticles prepared according to Preparation Example 1A in which the drug is a one-month dose of Finasteride.
FIG. 1b shows a particle size distribution graph of the microparticles prepared according to Preparation Example 1B in which the drug is a 3-month dose of Finasteride.
FIG. 2 shows a particle size distribution graph of the microparticles of the present disclosure prepared according to Preparation Example 2 in which the drug is a one-month dose of Donepezil.
FIG. 3 shows a particle size distribution graph of the microparticles of the present disclosure prepared according to Preparation Example 3 in which the drug is a one-month dose of Naltrexone.
FIG. 4 shows a particle size distribution graph of the microparticles of the present disclosure prepared according to Preparation Example 4 in which the drug is Exenatide.
FIG. 5 shows a particle size distribution graph of the microparticles of the present disclosure prepared according to Preparation Example 5 in which the drug is Leuprorelin.
FIG. 6 shows a particle size distribution graph of the microparticles of the present disclosure prepared according to Preparation Example 6 in which the drug is Deslorelin.
FIG. 7 shows a particle size distribution graph of the microparticles according to Comparative Example 1 in which the drug is Finasteride, but an emulsion solvent evaporation method is used.
FIG. 8 shows a particle size distribution graph of the microparticles according to Comparative Example 2 in which the drug is Donepezil, but the emulsion solvent evaporation method is used.
FIG. 9 shows a particle size distribution graph of Vivitrol^{®} (Alkermes plc), which is sold as a sustained-release injection product of Naltrexone, an alcohol addiction remedy drug.
FIG. 10 shows a particle size distribution graph of the microparticles prepared according to Comparative Example 4 in which the drug is Exenatide, but the emulsion solvent evaporation method is used.
FIG. 11 shows a particle size distribution graph of the microparticles prepared according to Comparative Example 5 in which the drug is Leuprorelin, but the emulsion solvent evaporation method is used.
FIG. 12 shows a particle size distribution graph of the microparticles prepared according to Comparative Example 6 in which the drug is Deslorelin, but the emulsion solvent evaporation method is used.
FIG. 13 shows a SEM (scanning electron microscope, ×500 magnification) photograph obtained by taking picture of the microparticles containing Finasteride, prepared according to Preparation Example 1A.
FIG. 14 shows SEM photographs obtained by taking picture of the microparticles containing Donepezil, prepared according to Preparation Example 2, at ×100, ×250, and ×950 magnifications.
FIG. 15A is a SEM photograph obtained by taking picture of the microparticles containing Naltrexone, prepared according to Preparation Example 3, at ×550 magnification, and FIG. 15B is a SEM photograph obtained by taking picture of Vivitrol^{®}, a commercially available product of Naltrexone, at × 500 magnification. The microparticles of the present disclosure exhibit a perfectly spherical shape, whereas the commercially available product exhibits an irregular shape with depressions and dents.
FIG. 16 is a graph showing the drug release experimental results of the microparticles containing Finasteride, prepared according to Preparation Example 1A of the present disclosure.
FIG. 17 is a table showing PK data for each period based on an animal experiment in which Injection product 1 is administered to a beagle dog to measure blood drug concentrations for each period.
FIG. 18 is PK and PD measurement results for the average blood concentration profile of Finasteride for each period and the change rate of DHT by an animal experiment in which Injection product 1 is administered to a beagle dog to measure blood drug concentrations for each period.
FIG. 19 is a graph showing the blood drug release experimental results for each period by administering Injection product 2 to a beagle dog.
FIG. 20 is a table showing PK data for each period based on an animal experiment in which Injection product 2 is administered to a beagle dog to measure blood drug concentrations for each period.
FIG. 21 is a graph showing the pharmacokinetic (PK) and pharmacodynamic (PD) analysis results of Injection product 2.
FIG. 22 shows a drug release graph of the microparticles containing Donepezil, prepared according to Preparation Example 2, in comparison with the drug release pattern of the control drug which is an oral dosage form.
FIG. 23 shows the drug release experimental results of the microparticles containing Naltrexone, prepared according to Preparation Example 3. As a comparison group, the release graph of Vivitrol^{®}, a commercially available injection product of Naltrexone, was presented together.

### Best Mode for carrying out the Invention

The present disclosure relates to sustained-release microparticles comprising a biodegradable polymer and a drug, wherein the biodegradable polymer and the drug are evenly distributed in the microparticles, the microparticles do not show an initial excessive release of the drug, and the microparticles exhibit a sustained release pattern of the drug over a desired period, in which the particle size distribution width analyzed by the particle size analyzer consists of a uniform particle size of 35 microns or less, and the specific surface area per unit mass of the particles is 0.75×10⁻¹ to 2.0×10⁻¹ m²/g.

### Modes for carrying out the Invention

Hereinafter, embodiments of the present disclosure will be described in detail so that those of ordinary skill in the art to which the present disclosure pertains can easily carry out the present disclosure. However, the present disclosure may be embodied in various different forms and is not limited to the embodiments described herein.

Microparticles containing a biodegradable polymer have already been well known in the field of sustained release formulations as spheres in the form of particulates for use in sustained release of a drug. Despite various attempts to continuously release the drug by encapsulating the drug in such a polymer carrier, in the case of a formulation in which the drug is encapsulated in microspheres made of the biodegradable polymer, a problem such as the initial excessive release of the drug or the inability to control the drug release caused by the non-uniformity of the particle size still exists.

The present disclosure provides microparticles as a sustained-release injection formulation of a drug, wherein the drug is uniformly encapsulated in the microparticles and the microparticles have controlled particle size characteristics, without being greatly influenced by variations in the type and content of the drug, the type and content of the biodegradable polymer, and the selected release period, so that the microparticles improve the completeness of the sustained release properties of the drug for a selected period without the initial excessive release of the drug. Further, also contemplated is a sustained-release injection composition comprising sustained-release microparticles that can reduce foreign body sensation and pain upon injection into a subject by formulating uniform and appropriately sized particles having a required drug concentration for a selected period of time into an injection product.

The sustained-release microparticles of the present disclosure have a biodegradable polymer and a drug uniformly distributed in the particles, and are monodisperse microspheres which have a smooth surface, a perfect spherical shape, a particle size distribution width of 35 microns or less, and a specific surface area of 0.75×10⁻¹ to 2.0×10⁻¹ m²/g.

The monodisperse microspheres have a perfectly spherical shape in which the biodegradable polymer and the drug are uniformly contained, and the surface thereof has an intact and smooth shape without flaws, depressions, protrusions, defects, or gaps.

That the drug is uniformly contained can be seen from the fact that the release of the drug from the prepared microparticles shows a sustained release pattern without initial excessive release.

The sustained-release microparticles of the present disclosure show that the release pattern of the drug from the particles has a ratio of initial blood concentration (Cᵢₙₜ) to maximum blood concentration (Cₘₐₓ) of 1:2 to 30 without initial excessive release.

In the present disclosure, "Cᵢₙₜ" refers to an initial blood concentration measurement value, and is a maximum blood concentration value measured within 24 hours.

This difference between the initial blood concentration value and the maximum blood concentration value means that no initial excessive release occurs. It also means that, due to the sustained release pattern, the maximum blood concentration value does not show a large difference with respect to the initial blood concentration value and maintains a constant range.

Further, the sustained-release microparticles of the present disclosure are ones in which the release pattern of the drug from the particles shows a sustained release pattern, and are composed of particles having almost the same particle diameter with a particle size distribution width of 35 microns or less, thereby showing a uniform pattern of drug release content over time. In particular, the preferred particle size distribution width in the sustained release microparticles of the present disclosure is 25 microns or less.

Further, the sustained-release microparticles of the present disclosure have a particle size distribution width of 35 microns or less and satisfy a specific surface area range of 0.75×10⁻¹ to 2.0×10⁻¹ m²/g. That is, even microparticles exhibiting particle size characteristics falling within the above specific surface area range are not included in the scope of the microparticles of the present disclosure unless the particle size distribution width is less than 35 microns.

In the sustained-release microparticles of the present disclosure, the biodegradable polymer is selected from the group consisting of polylactic acid, polylactide, polylactic-co-glycolic acid, polylactide-co-glycolide (PLGA), polyphosphazenes, polyiminocarbonates, polyphosphoesters, polyanhydrides, polyorthoesters, polycaprolactone, polyhydroxyvalerate, polyhydroxybutyrate, polyamino acids, and combinations thereof, and is particularly preferably selected from polylactide, polylactide-co-glycolide (PLGA), and a combination thereof. However, the present disclosure is not limited to these specific examples.

In the sustained-release microparticles of the present disclosure, the biodegradable polymer is in a range of 60 to 97% by weight based on the weight of the microparticles, and the drug is contained in a ratio range of 1:1.5 to 1:30 compared to the biodegradable polymer.

The preparation of the microparticles according to the present disclosure prepared using the biodegradable polymer and the drug proceeds in the order of: 1) a step (S100) of preparing a first mixture; 2) a step (S200) of preparing a second mixture; 3) a step (S300) of injecting the first mixture into a microchannel of the straight direction; 4) a step (S400) of injecting the second mixture into both sides or one side of the microchannel; 5) a step (S500) of collecting sustained-release particles; 6) a step (S600) of stirring the collected sustained-release particles; and 7) a step (S700) of washing and drying the sustained-release particles.

More specifically, a method for preparing sustained-release particles containing a drug according to an embodiment of the present disclosure will be described as follows.

The step of preparing the first mixture in the 1) step (S100) is a step of preparing a first mixture by dissolving a biodegradable polymer and a drug in an organic solvent, wherein the biodegradable polymer is selected from the group consisting of polylactic acid, polylactide, polylactic-co-glycolic acid, polylactide-co-glycolide (PLGA), polyphosphazenes, polyiminocarbonates, polyphosphoesters, polyanhydrides, polyorthoesters, polycaprolactone, polyhydroxyvalerate, polyhydroxybutyrate, polyamino acids, and combinations thereof, and is particularly preferably selected from polylactide, polylactide-co-glycolide (PLGA), and a combination thereof. However, the present disclosure is not limited to these specific examples.

Further, examples of the drug may include Donepezil, Aripiprazole, Olanzapine, Palonosetron, Minocycline, Memantine, Naltrexone, Alendronate, Deoxycholate, Risedronate, Ibandronate, Zoledronate, Liraglutide, Exenatide, Lanreotide, Octreotide, Deslorelin, Leuprorelin, Goserelin, Tryptorelin, and Finasteride, but the present disclosure is not particularly limited to these drugs only, and any drug that needs to be prepared as a sustained release type in the present technical field may be used in the present disclosure.

More specifically, Donepezil and Memantine for the prevention and treatment of dementia, Olanzapine and Aripiprazole for the prevention and treatment of psychosis, Palonosetron as an antiemetic, Minocycline as an antibiotic, Naltrexone for the prevention and treatment of alcoholism, Alendronate, Risedronate, Ibandronate, and Zoledronate for the treatment of osteoporosis, Deoxycholate, Liraglutide, and Exenatide for lipolytic dissolution and obesity treatment, Lanreotide and Octreotide for the treatment of acromegaly, Deslorelin, Goserelin, and Tryptorelin for the treatment of endometriosis, Leuprorelin for anticancer treatment, and Finasteride for the treatment of benign prostatic hyperplasia or hair loss may be used as drugs for the preparation of sustained-release microparticles.

In the step of preparing the first mixture in the 1) step (S100), when the drug is a water-soluble drug, the drug may be dissolved in water and used, but it is preferable to use an organic solvent that dissolves as much as possible. In this case, the drug and the biodegradable polymer may be dissolved in separate organic solvents, and the two solutions may be combined and used as the first mixture.

Further, in the step of preparing the first mixture in the 1) step (S100), the first mixture may be prepared by dissolving the drug and the biodegradable polymer in different suitable organic solvents respectively. As described above, if the drug and the biodegradable polymer may be prepared as a liquid or suspension using a medium capable of achieving an optimal dissolved or suspended state, the present disclosure is not significantly limited by the type of the solvent.

The biodegradable polymer and the drug in the first mixture may be adjusted to various ratios depending on the effective release dose of the drug, the required sustained administration period of the drug, physicochemical properties of the drug, etc. However, if the weight ratio of the biodegradable polymer compared to the drug is too small, it is not preferable since there is a problem in that the drug is not evenly distributed in the spherical biodegradable polymer particles, or the spherical particles are not formed. In general, the drug and the biodegradable polymer could be prepared up to the lowest content ratio of the drug to the biodegradable polymer of 1:1.5, but the present disclosure is not limited by this content ratio.

Further, since an effective amount of the drug cannot be incorporated into the particles of a desired size if the weight ratio of the biodegradable polymer compared to the drug is too large, there may be a problem in that a large amount of microparticles should be administered in order to administer the required drug concentration.

Therefore, the content ratio of the drug and the biodegradable polymer may be appropriately selected in consideration of the required dosage of the drug and the size of microparticles.

The biodegradable polymer is contained in the first mixture in an amount of about 10 to 30% by weight, preferably 10 to 20% by weight, and particularly preferably 12 to 15% by weight, but the present disclosure is not limited thereto.

Further, the organic solvent is an organic solvent capable of dissolving the biodegradable polymer and the drug, and any organic solvent that can be easily selected by those skilled in the art can be used. For example, it is one or more selected from the group consisting of dichloromethane, chloroform, chloroethane, dichloroethane, trichloroethane, and mixtures thereof, preferably dichloromethane, but the present disclosure is not limited to these examples.

The 2) step (S200) is a step of preparing a second mixture, and a surfactant is dissolved in water to prepare a second mixture. The surfactant is a component forming an aqueous phase as opposed to the first mixture, which is an oil phase, serves to break the oil phase solution in the form of droplets in the microchannel, and can be used without limitation as long as it can be used upon coagulation of these produced droplets. Specifically, it is one or more selected from the group consisting of nonionic surfactants, anionic surfactants, cationic surfactants, and mixtures of two or more thereof. More specifically, it is one or more selected from the group consisting of methylcellulose, polyvinylpyrrolidone, lecithin, gelatin, polyvinyl alcohol, polyoxyethylene sorbitan fatty acid ester, polyoxyethylene castor oil derivatives, sodium lauryl sulfate, sodium stearate, ester amine, linear diamine, fatty amine, and mixtures of two or more thereof. In particular, although polyvinyl alcohol (PVA) is preferable, the present disclosure is not limited to these examples.

The step 3) (S300) and the step (4) (S400) are steps of injecting the first mixture and the second mixture into the microchannels formed on the wafer respectively to allow them to flow.

More specifically, the microchannel may be formed in a material selected from the group consisting of a silicon wafer and a polymer film, but examples of the material are not limited to the above examples, and any material capable of forming microchannels can be used.

The polymer film may be selected from the group consisting of polyimide, polyethylene, fluorinated ethylene propylene, polypropylene, polyethylene terephthalate, polyethylene naphthalate, polysulfone, and mixtures thereof, but the present disclosure is not limited to the above examples.

For example, aluminum is deposited on a silicon wafer using an e-beam evaporator, and a photoresist is patterned on aluminum using a photolithography technique. After that, aluminum is etched using the photoresist as a mask, the photoresist is removed, silicon is etched by DRIE (deep ion reactive etching) using aluminum as a mask, aluminum is removed, and then glass is anodic bonded to be sealed on the wafer to manufacture the microchannel.

Further, the microchannel has an average diameter between 35 and 200 um, preferably 50 to 100 µm, but the present disclosure is not limited thereto. When the microchannel has an average diameter of 35 µm or less, there is a possibility that excessively small particles with a diameter of the microparticles prepared of 20 µm or less may be prepared. This increases the possibility that they are engulfed by macrophages after injection into the human body, which may affect the release of effective drugs and absorption thereof in vivo. Further, when the prepared particles have a size of more than 100 µm, the particles may block the injection needle during injection administration, and an injection needle with a large inner diameter should be used in order to prevent this, but in this case, the pain increases during injection, which is undesirable.

The average diameter of the microchannel is closely related to the average diameter of the particles, but is also closely related to the injection pressure of the first mixture and the second mixture so that it is not limited to the above examples, and may be changed depending on the average diameter of the particles to be prepared or the pressure conditions during injection.

Further, the cross-sectional width (w) and the cross-sectional height (d) of the microchannel are closely related to the average diameter (d') of microparticles to be prepared. The cross-sectional width (w) of the microchannel is in a ratio range of 0.7 to 2.3 with respect to the average diameter (d') of the microparticles, and the cross-sectional height (d) of the microchannel is in a ratio range of 0.7 to 2.3 with respect to the average diameter (d') of the particles.

That is, if the average diameter (d') of the particles to be prepared is determined, and accordingly, the length of the cross-sectional width (w) and cross-sectional height (d) of the microchannel should be set in a ratio range of 0.7 to 2.3 of d' so that particles having a desired size can be prepared.

That is, the first mixture flows along the microchannel of the straight direction, and the second mixture flows along the microchannel forming a point of intersection with the microchannel of the straight direction from both sides or one side based on the microchannel of the straight direction, and encounters with the flow of the first mixture.

At this time, when the first mixture is injected into the microchannel of the straight direction, it is injected under a constant pressure condition to allow it to flow at a constant flow rate, and the injection pressure condition of this organic phase is in a range of 200 to 1,700 mbar, preferably 500 to 800 mbar, but the present disclosure is not limited to these examples. Further, when the second mixture is injected into both sides or one side of the microchannel, it is injected under a constant pressure condition to allow it to flow at a constant flow rate, and the pressure condition at this time is in a range of 800 to 3,500 mbar, preferably 2,000 to 2,700 mbar, but the present disclosure is not limited to these examples.

As such, the second mixture is allowed to flow under higher pressure conditions such that the flow of the second mixture of the aqueous phase forming a point of intersection with the flow of the first mixture flows at a faster flow rate than the first mixture of the organic phase injected into the microchannel of the straight direction.

As described above, the second mixture having a relatively faster flow rate at a point where the flow of the first mixture encounters with the flow of the second mixture is allowed to compress the first mixture by differentiating the flow rates of the first mixture and the second mixture, and making the flow rate of the second mixture faster than that of the first mixture. At this time, the biodegradable polymer and drug in the first mixture produce spherical microparticles due to the repulsive force of the first mixture and the second mixture, and more specifically, microparticles having a form in which the drug is evenly distributed in a spherical biodegradable polymer are formed.

The step 5) (S500) is a step of collecting microparticles, and prevents agglomeration phenomenon between initially produced microparticles by collecting the microparticles in a water tank containing the second mixture.

The 5) step (S500) is one which uses the second mixture prepared in the second step (S200), that is, a mixed solution of a surfactant and water, and after the second mixture is prepared in the 2) step (S200), a portion of the second mixture is injected into the microchannel, and the other portion thereof is moved to the step 5) (S500) and used to prevent agglomeration phenomenon between the collected microparticles.

In this step, the concentration of the surfactant in the water tank may be the same as or different from the concentration in the second mixture. Moreover, it is preferable that the temperature in the water tank is a low temperature effective for particle formation. For example, the water tank may have a temperature of 15 to 25°C, preferably 17°C. Stirring may be performed in order to prevent agglomeration phenomenon of the particles collected in the water tank. The stirring is preferably performed to a stirring speed of about 150 to 650 rpm.

The step 6) (S600) is a step of stirring the microparticles collected in the water tank, and evaporates and removes the organic solvent present on the surface of the particles by stirring the microparticles at constant temperature conditions and stirring speed. At this time, the stirring may be performed by adjusting stirring conditions such as the temperature conditions and stirring speed required for the removal of the organic solvent and curing of the particles depending on the type of the drug and the type of the polymer.

As an example, the stirring may proceed in the order of: a step of performing first stirring at a speed of 150 to 650 rpm for 0.5 to 1.0 hour at 15 to 25°C; a step of performing second stirring at a speed of 200 to 1,000 rpm for 2.0 to 8.0 hours at 30 to 50°C after the first stirring step; and a step of performing third stirring at a speed of 200 to 1,000 rpm for 0.5 to 1.5 hours at 15 to 25°C after the second stirring step.

The stirring is performed by giving a difference in the stirring speed in the first and second stirring steps, thereby performing the stirring process using a faster speed in the second stirring process than in the first stirring process.

Also, in the temperature conditions in addition to the stirring speed, it is characterized in that stirring is performed by increasing the temperature in the second stirring process compared to the first stirring process, and as the temperature is increased step by step, the evaporation speed of the organic solvent present on the surface of the particles can be adjusted. That is, microparticles having a smooth surface can be prepared by slowly evaporating the organic solvent present on the surface of the sustained-release particles.

The temperature when the first mixture (oil phase) and the second mixture (aqueous phase) flow through the microchannel is 15 to 25°C, preferably 17°C. That is, after flowing through the microchannel and forming a point of intersection to produce microparticles, the temperature is constantly maintained at a low temperature of 15 to 25°C until the collected microparticles are subjected to first stirring. It is possible to prepare and maintain spherical particles only by maintaining a low temperature in the preparation process of the microparticles. That is, when it is not a low-temperature condition, there is a problem in that it is difficult to prepare particles having a constant spherical shape.

The mixture of the oil phase and the aqueous phase containing the microchannel formed at the point of intersection may be transferred into a tank containing a stock solution through a moving tubing having an inner diameter of 0.3 to 1.0 mm.

The stock solution may be an aqueous solution containing the same or the same type of surfactant as the surfactant used in the aqueous phase solution. The surfactant may have a content range of 0.2 to 1.0% by weight. The amount of the stock solution may be about 25 to 500 times that of the oil phase solution. The surfactant can be used without limitation as long as the biodegradable polymer solution can form a stable emulsion.

Finally, the step 7) (S700) is a step of washing and drying the microparticles. The microparticles from which all organic solvents on the surface have been removed by performing stirring are washed several times with sterilized filtered purified water to remove the surfactant remaining in the microparticles. Thereafter, the microparticles from which the surfactant has been removed are freeze-dried, dried under reduced pressure at room temperature, or rotationally dried at 35 to 45°C for 2 to 8 hours at 300 to 1500 rpm.

The weight ratio of the biodegradable polymer and drug contained in the finally produced particles is the same as the weight ratio in the first mixture, and this proves that, as the microparticles are prepared and the organic solvent is all removed by evaporation, the particles containing the biodegradable polymer and drug are prepared at the same ratio as the weight ratio in the first mixture. Therefore, the content of the biodegradable polymer present in the microparticles of the present disclosure is in a range of 60 to 97% by weight based on the weight of the microparticles. When the content of the biodegradable polymer is less than 60% by weight, it is difficult to form microspheres, and when the content of the biodegradable polymer is more than 97% by weight, it is not preferable since the release concentration of the drug becomes too small or it is difficult to increase the content of the drug without an increase in the size of the particles.

The finally obtained microparticles exhibit a particle distribution in which the size of the prepared particles is remarkably uniform compared to the size of the particles prepared by the solvent evaporation method publicly known in the present technical field. The microparticles prepared with the biodegradable polymer and drug according to the present disclosure are not significantly influenced by the type of the biodegradable polymer, the content or type of the drug, the mixing ratio of the biodegradable polymer and drug, the size of the produced particles, etc., and have a particle size distribution width of the total produced particles of 35 microns or less. In particular, the particle size distribution width is preferably 30 microns or less. More preferably, the sustained-release microparticles of the present disclosure have a particle size distribution of 25 microns or less.

In the case of preparing the microparticles using the solvent evaporation method, which is one of the publicly known methods for preparing microparticles, compared to the microparticles obtained through the biodegradable polymer and drug used in the present disclosure, microparticles having a particle size distribution width of as little as 1.5 times and as much as 2.5 times or more are produced. The solvent evaporation method generally comprises dissolving the biodegradable polymer and drug in an organic solvent to form an oil phase, putting the oil phase solution into an aqueous phase solution containing a surfactant and performing stirring to form a dispersed phase in the form of droplets, and heating the dispersed phase to a temperature of less than the boiling point temperature of the organic solvent to remove the solvent, thereby preparing microspheres. The particle size distribution width of the microspheres prepared by this method is as little as 55 microns or more and as much as 70 microns or more, showing a problem that the uniformity of the particles is greatly deteriorated. For polydisperse microspheres with non-uniform particle size, the release rate of the drug contained therein is inevitably different depending on the size of the particles, the problem of initial excessive release after administration may occur if there is a lot of formation of finer particle size, and such an excessive release of the drug may lead to the risk of the drug.

Further, the sustained-release microparticles of the present disclosure have a specific surface area between 0.75×10⁻¹ and 2.0 ×10⁻¹ m²/g. Preferably, the particles of the present disclosure have a specific surface area between 0.8×10⁻¹ and 1.7×10⁻¹ m²/g. In general, the specific surface area refers to the total surface area per unit mass or unit volume of the particles, and the particles respectively appear differently depending on the size or shape thereof. Accordingly, the sustained-release microparticles of the present disclosure have a particle size distribution width of 35 microns or less, and are composed of particles having a very narrow particle size distribution width, that is, monodisperse particles having a very uniform particle size. Therefore, even though the average particle diameter (D₅₀) is the same, since the microparticles of the present disclosure have a narrow particle size distribution width compared to particles having a particle size distribution width exceeding 35 microns, thereby having a large specific surface area and increasing the density of the particles per unit volume, they can also play a role in lowering the dosage of the drug as well as the uniform drug release.

Therefore, since the monodisperse particles having a particle size distribution width and specific surface area exhibited by the microparticles according to the present disclosure have a uniform particle size compared to the microparticles prepared by the solvent evaporation method so that the drug release pattern is inevitably constant and continuous by achieving uniformity and persistence of the dissolution rate and drug release rate of the biodegradable polymer upon injection into the patient, the problem of initial excessive release of the drug does not appear, and the effect of inducing continuous release of the drug for a desired period can be obtained through control of the content of the particles. Further, it is possible to induce a sustained release of the drug so that even if the dose of the drug is lowered, drug release that maintains the drug efficacy for a desired period can be achieved.

A composition for injection of a drug can be prepared using the microparticles of the present disclosure. The composition for injection containing the microparticles of the present disclosure may comprise drug-containing microparticles and a suspension solvent.

The composition for injection is a form in which microparticles are uniformly contained in the suspension solvent, and when the composition for injection is administered, the microparticles themselves may be injected into the body, thereby exhibiting the effect of long-term administration of the drug.

More specifically, when the microparticles of the present disclosure are injected into the body, the effect of releasing the drug by decomposition of the biodegradable polymer occurs, and at this time, since the particles of the present disclosure are in a form in which the biodegradable polymer and the drug are uniformly mixed, they may exhibit the effect of administering the drug at a constant concentration for a long time.

That is, at the time of one injection using the composition for injection of the present disclosure, the drug is continuously released from the body for a desired release period selected for 1 week to 12 months so that it may improve the convenience of users by solving the problem of having to take the drug every day.

The suspension solvent includes an isotonic agent, a suspending agent, and a solvent.

More specifically, the isotonic agent may be selected from the group consisting of D-mannitol, maltitol, sorbitol, lactitol, xylitol, sodium chloride, and mixtures thereof, and may preferably be D-mannitol, but the present disclosure is not limited to the above examples.

The suspending agent may be selected from the group consisting of sodium carboxymethyl cellulose, polysorbate 80, starch, starch derivatives, polyhydric alcohols, chitosan, chitosan derivatives, cellulose, cellulose derivatives, collagen, gelatin, hyaluronic acid (HA), alginic acid, algin, pectin, carrageenan, chondroitin, chondroitin sulfate, dextran, dextran sulfate, polylysine, titin, fibrin, agarose, fluran, xanthan gum, and mixtures thereof, and may preferably be sodium carboxymethyl cellulose and polysorbate 80, but the present disclosure is not limited to the above examples.

Injection water may be used as the solvent, and any solvent that can be used as injection water may be used without limitation.

Hereinafter, Examples of the present disclosure will be described in detail so that those skilled in the art to which the present disclosure pertains can easily carry out the present disclosure. However, the following Examples are illustrative of the present disclosure and do not limit the scope of the present disclosure.

### [Examples]

### Preparation Example: Preparation of Microparticles

### Preparation Example 1A

### Preparation of Microparticles containing one-month dose of Finasteride

A first mixture was prepared by dissolving 56.0 mg of polylactide-co-glycolide (PLGA) and 28.0 mg of Finasteride in 317 mg of dichloromethane (NF). At this time, polylactide-co-glycolide in the first mixture was contained at a ratio of 12.5% (w/w), and polylactide-co-glycolide and Finasteride were used at a weight ratio of 2:1.

48 mg of polyvinyl alcohol as a surfactant was mixed with water to prepare a second mixture containing 0.5% by weight of polyvinyl alcohol.

The first mixture and the second mixture were injected into a microchannel formed on a silicon wafer to flow. At this time, the microchannel used was a 120/80 gm orifice 7-channel chip. Further, in order to flow the first mixture and the second mixture at respectively different flow rates, the first mixture was flowed under a pressure condition of 450 mbar, and the second mixture was flowed under a pressure condition of 2,100 mbar. The temperature condition was maintained at 17°C.

The microparticles produced at the point of intersection where the flow of the first mixture and the flow of the second mixture met were moved along a tubing having an inner diameter of 0.5 mm so that they were collected in a water tank containing 0.25% by weight of an aqueous polyvinyl alcohol solution, and formation of the particles was completed while maintaining stirring of the microparticles collected in the water tank to 280 rpm at 17°C.

Thereafter, the formed microparticles were primarily stirred at a speed of 200 to 400 rpm for 1 hour at 17°C, secondly stirred at a speed of 300 to 800 rpm for 4 hours by raising the temperature to 43°C, and thirdly stirred at a speed of 200 to 1,000 rpm for 1 hour at 20°C so that the particles were cured and the organic solvent was removed.

The stirring-completed microparticles were washed several times with sterilized filtered purified water and freeze-dried to obtain sustained-release particles containing a one-month dose of Finasteride.

The diameter of the microparticles containing Finasteride obtained as described above was analyzed by a wet analysis method using a particle size analyzer of Microtac's S3500 model, in which a small amount of surfactant was dissolved in water as a dispersion, and the microparticles had an average diameter of 35.7 micrometers (µm), a diameter range of D10 %Tile 32.12 um to D90 %Tile 41.26 um, and a particle size distribution width of 7.24 um.

The results are as shown in Table 1 and FIG. 1a below.

### Preparation Example 1B

### Preparation of Microparticles containing a three-month dose of Finasteride

As a biodegradable polymer compound, a first mixture was prepared by dissolving a polymer mixture in which polylactide-co-glycolide (PLGA) and polylactide (PDL02A) had been mixed at a 1:1 ratio and 84 mg of Finasteride in dichloromethane. At this time, the polymer mixture in the first mixture was contained at a ratio of 12.5% (w/w), and the weight ratio of the polymer mixture and Finasteride was 2:1.

Polyvinyl alcohol as a surfactant was mixed with water to prepare a second mixture containing 0.5% by weight of polyvinyl alcohol.

The first mixture and the second mixture were injected into a microchannel formed on a silicon wafer to flow. At this time, the microchannel used was a 120/80 *µ*m orifice 7-channel chip. Further, in order to constantly flow the first mixture and the second mixture at respectively different flow rates, the first mixture was flowed under a pressure condition of 500 mbar, and the second mixture was flowed under a pressure condition of 2,100 mbar. The temperature condition was maintained at 17 °C.

The microparticles produced at the point of intersection where the flow of the first mixture and the flow of the second mixture met were moved along a tubing having an inner diameter of 0.5 mm so that they were collected in a water tank containing 0.25% by weight of an aqueous polyvinyl alcohol solution, and formation of the particles was completed while maintaining stirring of the microparticles collected in the water tank to 280 rpm at 17 °C.

Thereafter, the formed microparticles were primarily stirred at a speed of 200 to 400 rpm for 1 hour at 17°C, secondly stirred at a speed of 300 to 800 rpm for 5 hours by raising the temperature to 43°C, and thirdly stirred at a speed of 200 to 400 rpm for 1 hour at 25°C so that the particles were cured and the organic solvent was removed.

The stirring-completed microparticles were washed several times with sterilized filtered purified water and freeze-dried to obtain sustained-release particles containing a three-month dose of Finasteride.

The diameter of the microparticles containing Finasteride obtained as described above was analyzed by a wet analysis method using a particle size analyzer of Microtac's S3500 model, in which a small amount of surfactant was dissolved in water as a dispersion, and the microparticles had an average diameter of 36.42 micrometers (µm), a diameter range of D10 %Tile 32.46 um to D90 %Tile 41.76 um, and a particle size distribution width of 7.60 um.

The results are as shown in Table 1 and FIG. 1b below.

### Preparation Example 2

### Preparation of Microparticles containing Donepezil

A first mixture was prepared by dissolving a polymer mixture in which 4.5 g of polylactide (PDL02A) and 1.5 g of polylactide-co-glycolide (PDLG7502A) had been mixed at a 3:1 ratio and 1.34 g of Donepezil in 34 g of dichloromethane. At this time, the polymer mixture in the first mixture was contained at a ratio of 15.0% (w/w), and the weight ratio of the polymer mixture and Donepezil was 4.5:1.

Polyvinyl alcohol as a surfactant was mixed with water to prepare a second mixture containing 0.25% by weight of polyvinyl alcohol.

The first mixture and the second mixture were injected into a microchannel formed on a silicon wafer to flow. At this time, the microchannel used was a 120/80 gm orifice 7-channel chip. Further, in order to flow the first mixture and the second mixture at respectively different flow rates, the first mixture was flowed under a pressure condition of 1,000 mbar, and the second mixture was flowed under a pressure condition of 2,800 mbar. Through this, the flow rate ratio of the first mixture and the second mixture was adjusted to be kept constant. The temperature condition was maintained at 17 °C.

The microparticles produced at the point of intersection where the flow of the first mixture and the flow of the second mixture met were collected in a water tank containing 0.25% by weight of an aqueous polyvinyl alcohol solution, and during the preparation, the microparticles collected in the water tank were stirred to a speed of 300 rpm at 17 °C.

Thereafter, in order to stabilize and cure the collected microparticles, the microparticles were primarily stirred at a speed of 400 rpm for 1 hour at 17°C, and in order to completely remove the organic solvent used during preparation, the microparticles were secondly stirred at a speed of 600 rpm for 3 hours by raising the temperature to 40°C.

The stirring-completed microparticles were washed several times with sterilized filtered purified water, treated with a 4% mannitol solution as a cryoprotectant, and dried after freezing to obtain microparticles containing a one-month dose of Donepezil.

The diameter of the microparticles containing Donepezil obtained as described above was analyzed by a wet analysis method using a particle size analyzer of Microtac's S3500 model, in which a small amount of surfactant was dissolved in water as a dispersion, and the microparticles had an average diameter of 49.22 micrometers (µm), a diameter range of D10 %Tile 44.74 um to D90 %Tile 56.16 um, and a particle size distribution width of 8.69 um.

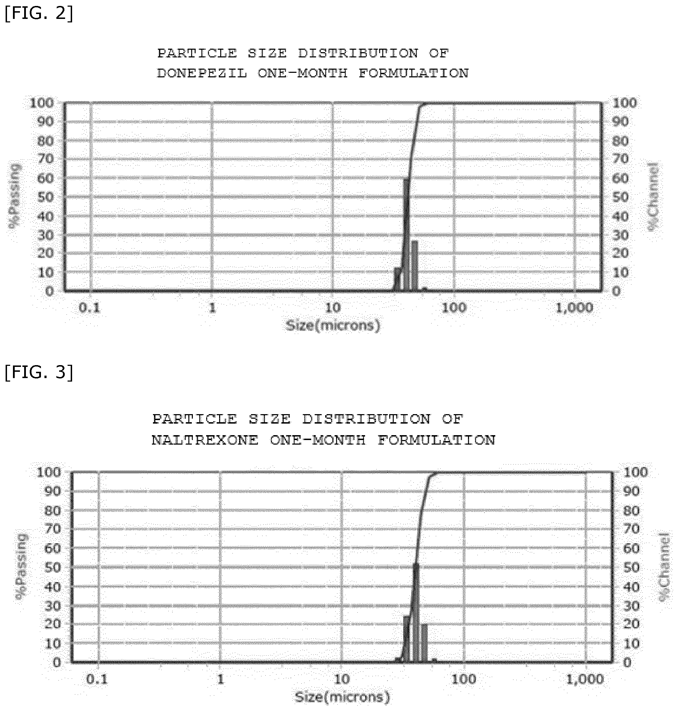

The results are as shown in Table 1 and FIG. 2 below.

### Preparation Example 3

### Preparation of Microparticles containing Naltrexone

After dissolving 1 g of Naltrexone in 2 g of benzyl alcohol and dissolving 2 g of polylactide-co-glycolide (PLGA 7504A) in 16 g of dichloromethane, the respective solutions were mixed to prepare a first mixture. At this time, the polymer mixture in the first mixture was contained at a ratio of 15.0% (w/w), and the polymer and Naltrexone were used at a weight ratio of 2:1.

Polyvinyl alcohol as a surfactant was mixed with water to prepare a second mixture containing 0.5% by weight of polyvinyl alcohol.

The first mixture and the second mixture were injected into a microchannel formed on a silicon wafer to flow. At this time, the microchannel used was a 100 *µ*m 7-channel chip. Further, in order to flow the first mixture and the second mixture at a constant flow rate, the first mixture was flowed under a pressure condition of 500 mbar, and the second mixture was flowed under a pressure condition of 2,500 mbar. The temperature condition was maintained at 17°C.

The microparticles produced at the point of intersection where the flow of the first mixture and the flow of the second mixture met were moved along a tubing having an inner diameter of 0.5 mm and collected in a water tank containing 0.5% by weight of an aqueous polyvinyl alcohol solution, and formation of the particles was completed while maintaining stirring of the microparticles collected in the water tank to 160 rpm at 17°C.

Thereafter, the formed microparticles were primarily stirred at a speed of 1,000 rpm for 1.5 hour at 15°C, secondly stirred at a speed of 1,000 rpm for 1 hour by raising the temperature to 20 °C, and then thirdly stirred at a speed of 1,000 rpm for 1 hour by raising the temperature to 25°C.

The stirring-completed microparticles were washed several times with sterilized filtered purified water, and freeze-dried to obtain microparticles containing a one-month dose of Naltrexone.

The diameter of the microparticles containing the one-month dose of Naltrexone obtained as described above was analyzed by a wet analysis method using a particle size analyzer of Microtac's S3500 model, in which a small amount of surfactant was dissolved in water as a dispersion, and as a result, the microparticles had an average diameter of 40.01 micrometers (µm), a diameter range of D10 %Tile 33.73 um to D90 %Tile 47.46 um, and a particle size distribution width of 10.38 um.

The results are as shown in Table 1 and FIG. 3 below.

### Preparation Example 4

### Preparation of Microparticles containing Exenatide

After dissolving 0.1 g of Exenatide in 8 g of dimethyl sulfoxide and dissolving 2.4 g of a polymer mixture in which polylactide and polylactide-co-glycolide (PLGA) had been mixed at a ratio of 3:1 in 13.6 g of dichloromethane, the two solutions were mixed to prepare a first mixture. At this time, the polymer mixture in the first mixture was contained at a ratio of 15.0% (w/w), and the polymer mixture and Exenatide were used at a weight ratio of 24:1.

Polyvinyl alcohol as a surfactant was mixed with water to prepare a second mixture containing 0.5% by weight of polyvinyl alcohol.

The first mixture and the second mixture were injected into a microchannel formed on a silicon wafer to flow. At this time, in order to flow the first mixture and the second mixture at a constant flow rate, the first mixture was flowed under a pressure condition of 1,500 mbar, and the second mixture was flowed under a pressure condition of 2,700 mbar. The temperature condition was maintained at 17°C.

The microparticles produced at the point of intersection where the flow of the first mixture and the flow of the second mixture met were moved along a tubing having an inner diameter of 0.5 mm and collected in a water tank containing 0.5% by weight of an aqueous polyvinyl alcohol solution, and formation of the particles was completed by maintaining stirring of the collected microparticles to a speed of 350 rpm for 1 hour at 17°C.

Thereafter, the formed microparticles were primarily stirred at a speed of 200 to 400 rpm for 1 hour at 17°C, secondly stirred at a speed of 300 to 800 rpm for 4 hours by raising the temperature to 40°C, and thirdly stirred at a speed of 200 to 1,000 rpm for 1 hour at 20°C so that the particles were cured and the organic solvent was removed.

The stirring-completed microparticles were washed several times with sterilized filtered purified water, and freeze-dried to obtain sustained-release particles containing a one-month dose of Exenatide.

The diameter of the microparticles containing Exenatide obtained as described above was analyzed by a wet analysis method using a particle size analyzer of Microtac's S3500 model, in which a small amount of surfactant was dissolved in water as a dispersion, and the microparticles had an average diameter of 48.08 micrometers (µm), a diameter range of D10 %Tile 41.36 µm to D90 %Tile 55.36 µm, and a particle size distribution width of 10.23 um.

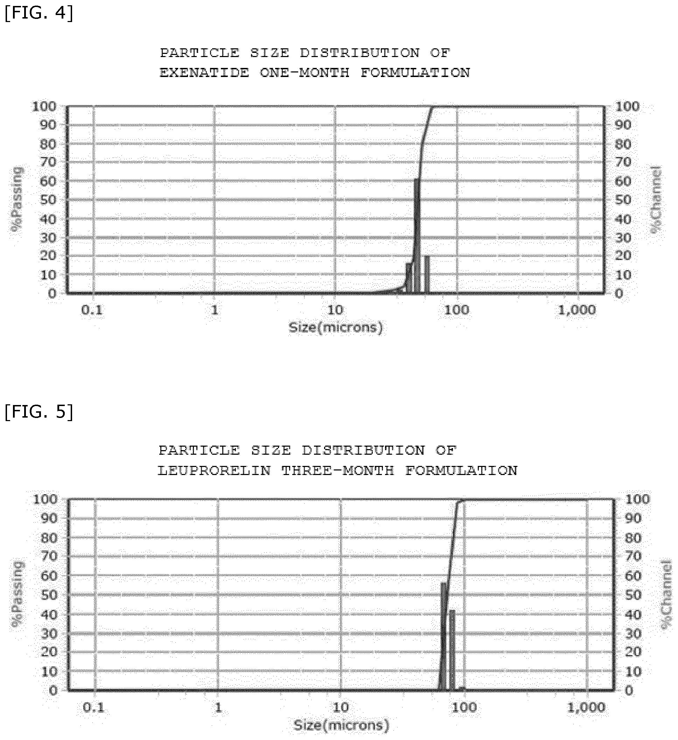

The results are as shown in Table 1 and FIG. 4 below.

### Preparation Example 5

### Preparation of Microparticles containing a three-month dose of Leuprorelin

After dissolving 0.2 g of Leuprorelin in 1.0 g of dimethyl sulfoxide and dissolving 1.0 g of polylactide (PDL02A) in 3.75 g of dichloromethane (DCM), the two solutions were mixed to prepare a first mixture. At this time, polylactide in the first mixture was contained at a ratio of 15.0% (w/w), and polylactide and Leuprorelin were used at a weight ratio of 5:1.

Polyvinyl alcohol as a surfactant was mixed with water to prepare a second mixture containing 0.5% by weight of polyvinyl alcohol.

The first mixture and the second mixture were injected into a microchannel formed on a silicon wafer to flow. At this time, the microchannel used was a 100 *µ*m 7-channel chip. Further, in order to flow the first mixture and the second mixture at respectively different flow rates, the first mixture was flowed under a pressure condition of 1,500 mbar, and the second mixture was flowed under a pressure condition of 2,700 mbar. The temperature condition was maintained at 17 °C.

The microparticles produced at the point of intersection where the flow of the first mixture and the flow of the second mixture met were moved along a tubing having an inner diameter of 0.5 mm and collected in a water tank containing 0.5% by weight of an aqueous polyvinyl alcohol solution, and formation of the particles was completed while maintaining stirring of the microparticles collected in the water tank to a speed of 300 rpm for 1 hour at 17 °C.

Thereafter, the formed microparticles were primarily stirred at a speed of 200 to 400 rpm for 1 hour at 17°C, secondly stirred at a speed of 300 to 800 rpm for 4 hours by raising the temperature to 43°C, and thirdly stirred at a speed of 200 to 1,000 rpm for 1 hour at 20°C so that the particles were cured and the organic solvent was removed.

The stirring-completed microparticles were washed several times with sterilized filtered purified water, and freeze-dried to obtain microparticles containing a three-month dose of Leuprorelin.

The diameter of the microparticles containing Leuprorelin obtained as described above was analyzed by a wet analysis method using a particle size analyzer of Microtac's S3500 model, in which a small amount of surfactant was dissolved in water as a dispersion, and the microparticles had an average diameter of 72.69 µm, a diameter range of D10 %Tile 64.87 µm to D90 %Tile 83.70 µm, and a particle size distribution width of 15.33 um.

The particle measurement results of the microparticles are as shown in Table 1 and FIG. 5 below.

### Preparation Example 6

### Preparation of Microparticles containing a six-month dose of Deslorelin

After dissolving 0.3 g of Deslorelin in 1.0 g of methyl alcohol and dissolving 2.0 g of polylactide (PDL02A) in 4.6 g of dichloromethane, the two solutions were mixed to prepare a first mixture. At this time, the polymer mixture in the first mixture was contained at a ratio of 15.0% (w/w), and the weight ratio of the polymer and Deslorelin was about 6.7:1.

Polyvinyl alcohol as a surfactant was mixed with water to prepare a second mixture containing 0.5% by weight of polyvinyl alcohol.

The first mixture and the second mixture were injected into a microchannel formed on a silicon wafer to flow. At this time, the microchannel used was a 100 *µ*m 7-channel chip. Further, in order to flow the first mixture and the second mixture at respectively different flow rates, the first mixture was flowed under a pressure condition of 1,700 mbar, and the second mixture was flowed under a pressure condition of 2,700 mbar. The temperature condition was maintained at 17°C.

The microparticles produced at the point of intersection where the flow of the first mixture and the flow of the second mixture met were moved along a tubing having an inner diameter of 0.5 mm and collected in a water tank containing 0.5% by weight of an aqueous polyvinyl alcohol solution, and formation of the particles was completed by maintaining stirring of the microparticles collected in the water tank to a speed of 400 rpm for 1 hour at 17 °C.

Thereafter, the collected microparticles were primarily stirred at a speed of 200 to 400 rpm for 1 hour at 17°C, secondly stirred at a speed of 300 to 800 rpm for 4 hours by raising the temperature to 43°C, and thirdly stirred at a speed of 200 to 1,000 rpm for 1 hour at 20°C.

The stirring-completed microparticles were washed several times with sterilized filtered purified water, and freeze-dried to obtain microparticles containing a six-month dose of Deslorelin.

The diameter of the microparticles containing Deslorelin obtained as described above was analyzed by a wet analysis method using a particle size analyzer of Microtac's S3500 model, in which a small amount of surfactant was dissolved in water as a dispersion, and the microparticles had an average diameter of 73.05 µm, a diameter range of D10 %Tile 65.02 µm to D90 %Tile 85.87 µm, and a particle size distribution width of 15.27 um.

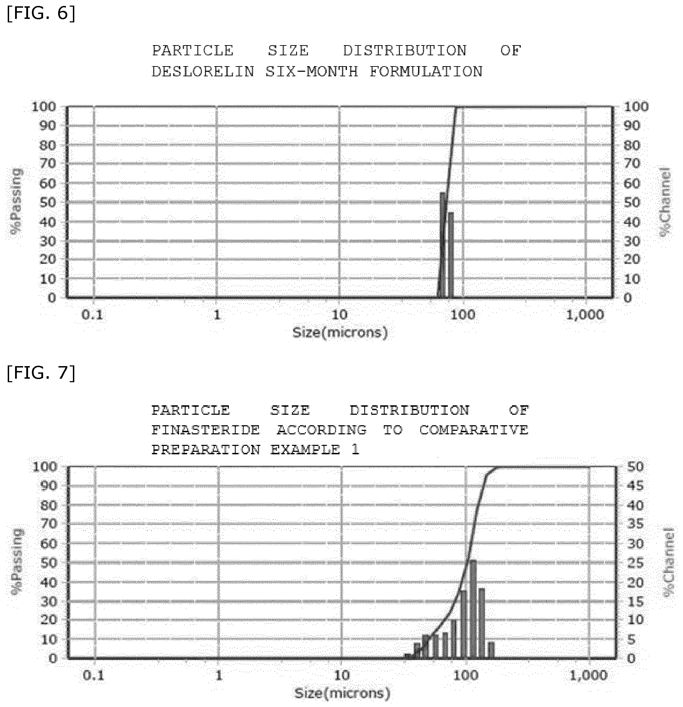

The particle measurement results of the microparticles are as shown in Table 1 and FIG. 6 below.

**[Table 1]**

| | Preparation Example (Present Disclosure) | | | | | | |
|---|---|---|---|---|---|---|---|
| | Preparation Example 1A Finasteride (1M) | Preparation Example 1B Finasteride (3M) | Preparation Example 2 Donepezil (1M) | Preparation Example 3 Naltrexone (1M) | Preparation Example 4 Exenatide (1M) | Preparation Example 5 Leuprorelin (3M) | Preparation Example 6 Deslorelin (6M) |
| Drug content (mg) | 28 | 84 | 63.84 | 100 | 100 | 200 | 300 |
| Polymer content (mg) | 56 | 168 | 287.28 | 200 | 2,400 | 1,000 | 2,200 |
| Drug : Polymer | 1:2 | 1:2 | 1:4.5 | 1:2 | 1:24 | 1:5 | 1:6.7 |

| %Tile | Particle size (*µ*m) | | | | | | |
|---|---|---|---|---|---|---|---|
| 10.00 | 32.12 | 32.46 | 44.74 | 33.73 | 41.36 | 64.87 | 65.02 |
| 20.00 | 33.09 | 33.50 | 46.05 | 35.86 | 44.13 | 66.92 | 67.13 |
| 30.00 | 33.98 | 34.48 | 47.10 | 37.47 | 45.56 | 68.85 | 69.12 |
| 40.00 | 34.83 | 35.43 | 48.16 | 38.78 | 46.85 | 70.71 | 71.04 |
| 50.00 | 35.70 | 36.42 | 49.22 | 40.01 | 48.08 | 72.69 | 73.05 |
| 60.00 | 36.66 | 37.49 | 50.31 | 41.30 | 49.36 | 74.82 | 75.22 |
| 70.00 | 37.84 | 38.68 | 51.59 | 42.70 | 50.75 | 77.23 | 77.58 |
| 80.00 | 39.30 | 40.06 | 53.31 | 44.46 | 52.32 | 80.12 | 80.29 |
| 90.00 | 41.26 | 41.76 | 56.16 | 47.46 | 55.36 | 83.70 | 83.63 |
| 95.00 | 42.59 | 42.90 | 58.57 | 50.03 | 57.88 | 86.18 | 85.87 |
| Particle size distribut ion width | 7.24 | 7.6 | 8.69 | 10.38 | 10.23 | 15.33 | 15.27 |
| Span value | 0.256 | 0.255 | 0.232 | 0.343 | 0.291 | 0.259 | 0.255 |
| Specific surface area (m²/g) | 1.67×10⁻¹ | - | 1.21×10⁻¹ | 1.43×10⁻¹ | 1.28×10⁻¹ | 0.82×10⁻¹ | 0.82×10⁻¹ |

As can be seen from the particle analysis results, it was confirmed that the microparticles of the present disclosure prepared in Preparation Examples 1A, 1B and 2 to 6 above were prepared as uniformly sized particles without being largely dependent on the compositions and contents of various biodegradable polymers, the types and contents of drugs, and the blending ratios of polymers and drugs. Specifically, the microparticles have an average diameter (D₅₀) of 35.70 microns (Preparation Example 1A) to 73.05 microns (Preparation Example 6), and it can be confirmed that the particle diameter is distributed in the range of 20 to 100 microns. The microparticles are uniformly sized microparticles having a particle size distribution width of 35 microns or less, and have a specific surface area of 0.75×10⁻¹ to 2.0×10⁻¹ m²/g.

In Comparative Examples below, particles were prepared using the solvent evaporation method, which is one of the general methods for preparing sustained-release microspheres, and properties of the particles were checked and compared with those of the microparticles of the present disclosure.

### Comparative Example 1

### Preparation of Microparticles containing Finasteride using Solvent Evaporation Method

Finasteride and biodegradable polymer were mixed in the same content and composition ratio as in Preparation Example 1, and particles were prepared by injecting the mixture into a 0.5% aqueous polyvinyl alcohol solution using a syringe having a needle gauge of 16G (inner diameter of 1.19 mm). The injection rate of 0.15 ml/sec was used. At this time, high-speed stirring was performed under the stirring conditions of 17°C and 2,000 rpm to form a dispersed phase in the form of droplets, it was solidified at a temperature of 20°C for 3 hours, and then microparticles were formed by removing the dichloromethane solvent while performing stirring at a temperature of 35°C for 1 hour. The microparticles were washed three times with water to remove polyvinyl alcohol.

The diameter of the microparticles containing Finasteride obtained as described above was analyzed in the same manner using the same particle size analyzer as in Preparation Examples of the present disclosure above, and as a result, the microparticles had an average diameter (D₅₀) of 103.1 micrometers (*µ*m), a diameter range of D10 %Tile 50.68 µm to D90 %Tile 138.5 µm, and a particle size distribution width of 70.94 µm.

The measurement results of the particle properties of the microparticles are as shown in Table 2 and FIG. 7 below.

### Comparative Example 2

### Preparation of Microparticles containing Donepezil using Solvent Evaporation Method

Donepezil and biodegradable polymer were mixed in the same content and composition ratio as in Preparation Example 2, and particles were prepared by injecting the mixture into a 0.5% aqueous polyvinyl alcohol solution using a syringe having a needle gauge of 16G (inner diameter of 1.19 mm). The injection rate of 0.15 ml/sec was used. At this time, high-speed stirring was performed under the stirring conditions of 17°C and 2,000 rpm to form a dispersed phase in the form of droplets, it was solidified at a temperature of 20°C for 3 hours, and then microparticles were formed by removing the dichloromethane solvent while performing stirring at a temperature of 35°C for 1 hour. The microparticles were washed three times with water to remove polyvinyl alcohol.

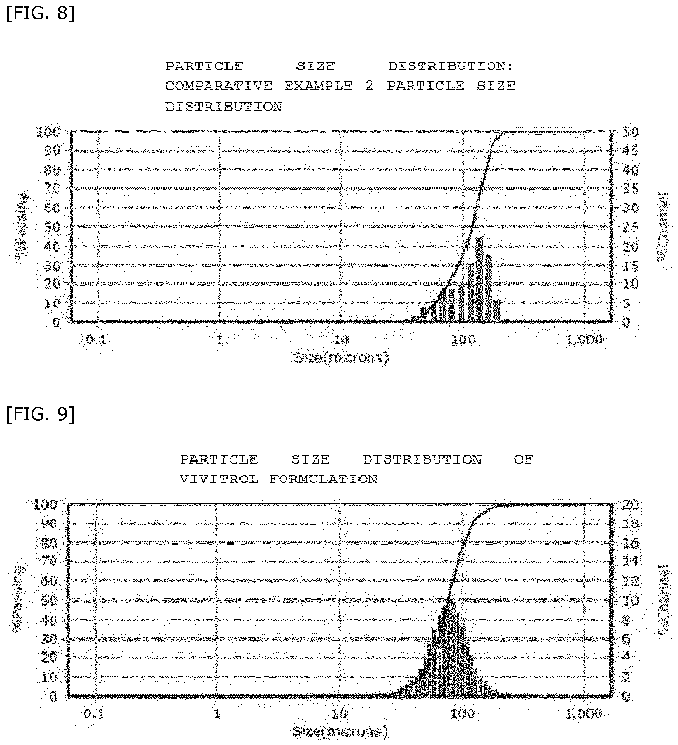

The diameter of the Donepezil microparticles obtained by the solvent evaporation method as described above was analyzed in the same manner using the same particle size analyzer as in Preparation Examples of the present disclosure above, and as a result, the microparticles had an average diameter (D₅₀) of 120.2 micrometers (*µ*m), a diameter range of D10 %Tile 59.48 µm to D90 %Tile 167.9 µ m, and a particle size distribution width of 120.2 µm. The measurement results of the particle properties of the microparticles are as shown in Table 2 and FIG. 8 below.

### Comparative Example 3

### Preparation of Vivitrol Microparticles, Commercial Long-Term Sustained Release Formulation of Naltrexone

A sustained-release formulation containing Naltrexone, currently sold under the trade name Vivitrol^{®} (manufacturer Alkermes pic), was purchased and the particle properties were analyzed using the same apparatus and method as in Preparation Examples of the present disclosure. As a result, the microparticles had an average diameter of 77.05 micrometers (µm), a diameter range of D10 %Tile 46.40 um to D90 %Tile 122.2 um, and a particle size distribution width of 55.98 um.

The measurement results of the particle properties of the microparticles are as shown in Table 2 and FIG. 9 below.

### Comparative Example 4

### Preparation of Microparticles containing Exenatide using Solvent Evaporation Method

Exenatide and biodegradable polymer were mixed in the same content and composition ratio as in Preparation Example 4, and particles were prepared by injecting the mixture into a 0.5% aqueous polyvinyl alcohol solution using a syringe having a needle gauge of 16G (inner diameter of 1.19 mm). The injection rate of 0.15 ml/sec was used. At this time, high-speed stirring was performed under the stirring conditions of 17°C and 2,000 rpm to form a dispersed phase in the form of droplets, it was solidified at a temperature of 20°C for 3 hours, and then microparticles were formed by removing the dichloromethane solvent while performing stirring at a temperature of 35°C for 1 hour. The microparticles were washed three times with water to remove polyvinyl alcohol.

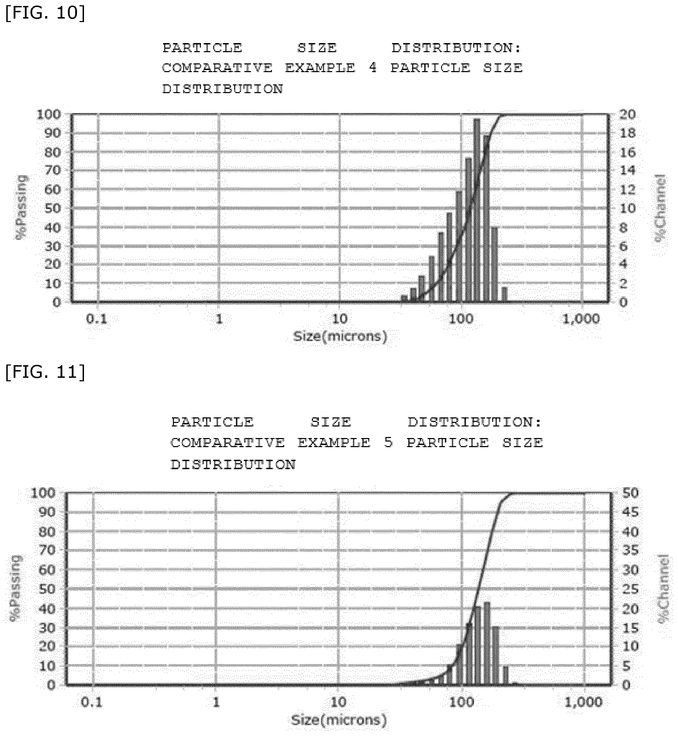

The diameter of the Exenatide microparticles obtained by the solvent evaporation method as described above was analyzed in the same manner using the same particle size analyzer as in Preparation Examples of the present disclosure above, and as a result, the microparticles had an average diameter (D₅₀) of 120.2 *µ*m, a diameter range of D10 %Tile 62.92 µm to D90 %Tile 174.9 µm, and a particle size distribution width of 91.15 µm. The measurement results of the particle properties of the microparticles are as shown in Table 2 and FIG. 10 below.

### Comparative Example 5

### Preparation of Microparticles containing Leuprorelin using Emulsion Solvent Evaporation Method

Leuprorelin and biodegradable polymer were mixed in the same content and composition ratio as in Preparation Example 5, and particles were prepared by injecting the mixture into a 0.5% aqueous polyvinyl alcohol solution using a syringe having a needle gauge of 16G (inner diameter of 1.19 mm). The injection rate of 0.15 ml/sec was used. At this time, high-speed stirring was performed under the stirring conditions of 17°C and 2,000 rpm to form a dispersed phase in the form of droplets, it was solidified at a temperature of 20°C for 3 hours, and then microparticles were formed by removing the dichloromethane solvent while performing stirring at a temperature of 35°C for 1 hour. The microparticles were washed three times with water to remove polyvinyl alcohol.

The diameter of the Leuprorelin microparticles obtained by the emulsion solvent evaporation method as described above was analyzed in the same manner using the same particle size analyzer as in Preparation Examples of the present disclosure above, and as a result, the microparticles had an average diameter (D₅₀) of 122.4 *µ*m, a diameter range of D10 %Tile 64.08 µm to D90 %Tile 176.3 µm, and a particle size distribution width of 91.61 µm. The measurement results of the particle properties of the microparticles are as shown in Table 2 and FIG. 11 below.

**[Table 2]**

| %Tile | Comparative Example 1 (Finasteride) (*µ*m) | Comparativ e Example 2 (Donepezil ) (*µ*m) | Comparative Example 3 (Vivitrol^{®}) (*µ*m) | Comparative Example 4 (Exenatide) (*µ*m) | Comparativ e Example 5 (Leuprorel (*µ*m) in) | Comparative Example 6 (Deslorelin) |
|---|---|---|---|---|---|---|
| 10.00 | 50.68 | 59.48 | 46.40 | 62.92 | 84.63 | 64.08 |
| 20.00 | 67.14 | 74.50 | 56.61 | 78.66 | 101.7 | 79.94 |
| 30.00 | 83.10 | 90.86 | 63.94 | 93.21 | 115.1 | 94.81 |
| 40.00 | 94.30 | 106.8 | 70.52 | 107.2 | 127.1 | 109.1 |
| 50.00 | 103.1 | 120.2 | 77.05 | 120.2 | 138.6 | 122.4 |
| 60.00 | 110.8 | 131.1 | 84.01 | 132.3 | 150.3 | 134.3 |
| 70.00 | 118.3 | 141.3 | 92.24 | 144.3 | 162.6 | 146.4 |
| 80.00 | 126.9 | 152.9 | 103.2 | 157.7 | 176.7 | 159.5 |
| 90.00 | 138.5 | 167.9 | 122.2 | 174.9 | 196.0 | 176.3 |
| 95.00 | 147.1 | 180.2 | 205.6 | 189.6 | 210.8 | 190.7 |
| Particle size distrib ution width (*µ*m) | 70.94 | 89.9 | 55.98 | 91.15 | 87.75 | 91.61 |
| Span value | 0.852 | 0.902 | 0.984 | 0.932 | 0.804 | 0.917 |
| Specific surface area | 0.7x10⁻¹ | 0.6x10⁻¹ | 0.87x10⁻¹ | 0.59x10⁻¹ | 0.49x10⁻¹ | 0.58x10⁻¹ |

As shown in Table 2, the microparticles having the average diameter of 103.1 um of Comparative Example 1 prepared by the solvent evaporation method using Finasteride appear to be prepared to a size reaching about 2.89 times that of the present disclosure (35.70 um). Besides, the particle size distribution width (70.94 um) of Comparative Example 1 was also in a range of about 10 times the particle size distribution width (7.24 um) of the present disclosure. If the Span value (D(0.9)-D(0.1)/D(0.5)) meaning the width of the particle size distribution is usually 0.5 or less, the particle size distribution may be determined to be uniform. The particles prepared in Comparative Example 1 have a Span value of 0.852, and the Finasteride particles of the present disclosure have a Span value of 0.256, which proves that the size of the particles prepared in Comparative Example 1 is not very uniform.

Further, according to Table 2, the microparticles having the average diameter of 120.2 um of Comparative Example 2 prepared by the solvent evaporation method using Donepezil appear to be prepared to a size reaching about 2.44 times that of the present disclosure (49.22 um). Besides, the particle size distribution width (89.9 um) of Comparative Example 2 was also in a range of about 10.35 times the particle size distribution width (8.69 um) of the present disclosure. If the Span value (D(0.9)-D(0.1)/D(0.5)) meaning the width of the particle size distribution is usually 0.5 or less, the particle size distribution may be determined to be uniform. The particles prepared in Comparative Example 2 have a Span value of 0.902, and the Donepezil microparticles of the present disclosure have a Span value of 0.255, which proves that the size of the particles prepared in Comparative Example 2 is not very uniform.

The microparticles of the drugs of Comparative Examples 3 to 6 also exhibited remarkably larger average particle diameters, particle size distribution widths, and Span values than the microparticles according to the present disclosure, suggesting the nonuniformity of the particles. In particular, the commercial formulation of Naltrexone of Comparative Example 3 was shown to have an average particle diameter of 77.05 um and a particle size distribution width of 55.98 um.

That is, the commercial formulation of Naltrexone had an average particle diameter of about 1.5 times or more larger than that of the Naltrexone-containing microparticles of the present disclosure of 40.01 micrometers (gm), and also had a particle size distribution width of about 5 times or more larger than that of the present disclosure of 10.38 µm. Further, the particles of Comparative Example 3 (0.87×10⁻¹) showed a large difference also in specific surface area compared to the microparticles of the present disclosure (Preparation Example 3 (1.43×10⁻¹)).

In order to confirm the drug release pattern for the microparticles of the present disclosure composed of uniform particles having a small particle diameter and a small particle size distribution width, the microparticles containing Finasteride, Donepezil, and Naltrexone were each prepared as a composition for injection so that the animal test was performed.

### Preparation of Composition for Injection

### Injection product 1: Preparation of 1M Composition for Injection containing a One-month Dose of Finasteride

The microparticles of Preparation Example 1A containing 28 mg of Finasteride as a one-month dose were added to 2 mL of a suspension solvent and uniformly suspended to prepare a 1M injection formulation containing Finasteride.

The suspension solvent used is composed of the composition as shown in Table 3 below.

**[Table 3]**

| **Content criterion** | **Purpose of blending** | **Ingredient name** | **Amount** | **Unit** |
|---|---|---|---|---|
| 2.0 mL | Isotonic agent | D-Mannitol | 100.0 | mg |
| | Suspending agent | Sodium Carboxymethyl cellulose | 10.0 | mg |
| | Suspending agent | Polysorbate 80 | 2.0 | mg |
| | Solvent | Water for injection | Suitable amount | |

### Injection Product 2: Preparation of 3M Composition for Injection containing a Three-month Dose of Finasteride

The microparticles of Preparation Example 1B containing 84 mg of Finasteride as a three-month dose were added to 2 mL of a suspension solvent and uniformly suspended to prepare a 3M injection formulation containing Finasteride.

### Injection Product 3: Preparation of Composition for Injection containing a One-month Dose of Donepezil

The microparticles containing Donepezil of Preparation Example 2 were added to 2 mL of the suspension solvent of Table 3 above and uniformly suspended to prepare a 1M injection formulation containing Donepezil.

### Injection Product 4: Preparation of Composition for Injection containing a One-month Dose of Naltrexone

The microparticles containing Naltrexone of Preparation Example 3 were added to 2 mL of the suspension solvent of Table 3 above and uniformly suspended to prepare a 1M injection formulation containing Naltrexone.

### Evaluation of Appearance and Release Properties of Microparticles

### Test Example 1: Examination of Properties of Sustained-Release Particles

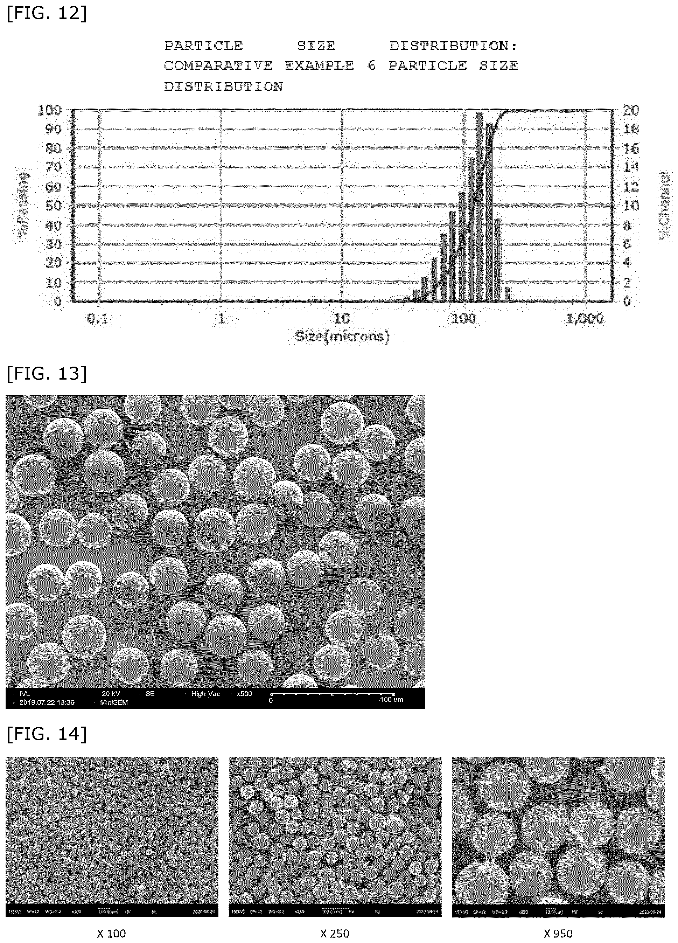

In order to confirm the properties of the microparticles, the shapes of the particles were confirmed by performing SEM photographing of the sustained-release particles obtained according to Preparation Examples. As a result, it could be confirmed that all of them were perfectly spherical. The SEM photograph of the Finasteride microparticles is shown in FIG. 13, and the SEM photographs of the microparticles containing Donepezil are shown in FIG. 14.

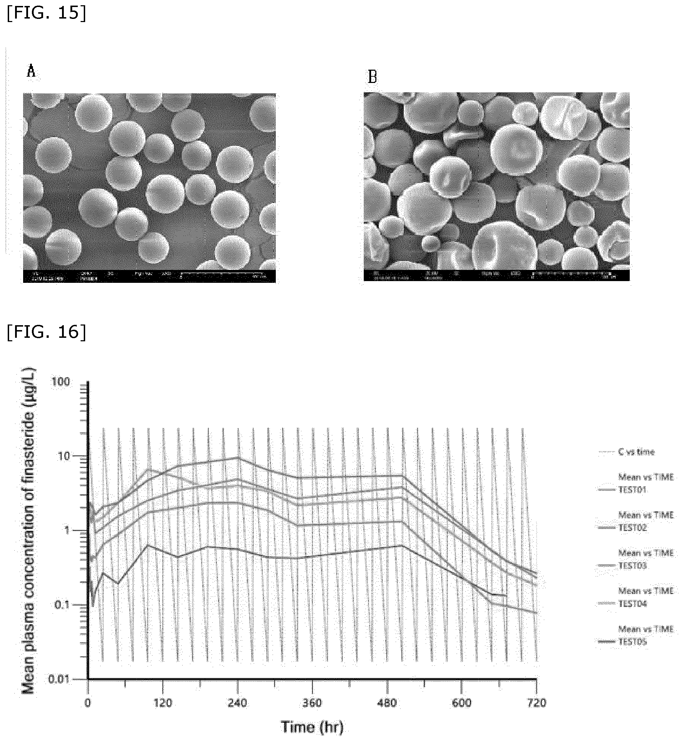

Further, the SEM photograph of the Naltrexone sustained-release particles prepared according to Preparation Example 3 and the SEM photograph of Vivitrol^{®} (Alkermes, inc), a commercially available injection product of Naltrexone as a comparison group, were compared to be shown in FIG. 15. FIG. 15A shows a photograph taking picture of the Naltrexone microparticles of the present disclosure at ×550 magnification, and it can be confirmed that perfectly spherical sustained-release particles without flaws on the surface are produced, whereas it can be confirmed in the SEM photograph (×500) of Vivitrol^{®} particles for comparison that a depression is present in the sphere as shown in FIG. 15B.

### Test Example 2: Release Profile of Microparticles (In vivo Release Experiment)

### Test Example 2-1. Finasteride Drug Release

The degree of drug release for the prepared microparticles was evaluated. Whether it could be used as a sustained release formulation or not was confirmed by evaluating the release pattern of the drug over time.

After a single administration of the composition for injection of Composition Preparation Example 1 above to a beagle dog, the concentration of Finasteride in blood was quantified using LC-MS/MS.

Pharmacokinetics (PKs) analysis was performed using WinNonlin^{®} software (version 8.0, Pharsight^{®}, Certara^{™} Company). In analysis methods, and main instruments and devices used in the analysis, the analysis may be performed using instruments conventional in the present technical field, and those skilled in the art will be able to fully understand.

The collected dissolution sample was analyzed for the content of each drug using HPLC, and the dissolution rate was calculated. A graph of drug release of the microparticles containing Finasteride is shown in FIG. 16. In the graph shown in FIG. 16, the five drugs show a uniform sustained release pattern without exhibiting an initial excessive release regardless of the dosing rates, as release patterns according to dosing rates of the microparticles (10% (Test01), 20% (Test02), 40% (Test03), 60% (Test04), and 100% (Test05)). As a control group, the release pattern of the oral dosage form is indicated by a black line.

Further, the PK parameters of the test drug administration group calculated area under the plasma concentration-time curve from time zero to t (AUC₀₋ₜ), mean (Mean), standard deviation (SD), and maximum blood concentration (Cₘₐₓ) (µg/L) using 'WinNonlin^{®} program (ver. 8.0, Pharsight^{®}, a Certara^{™} company)'.

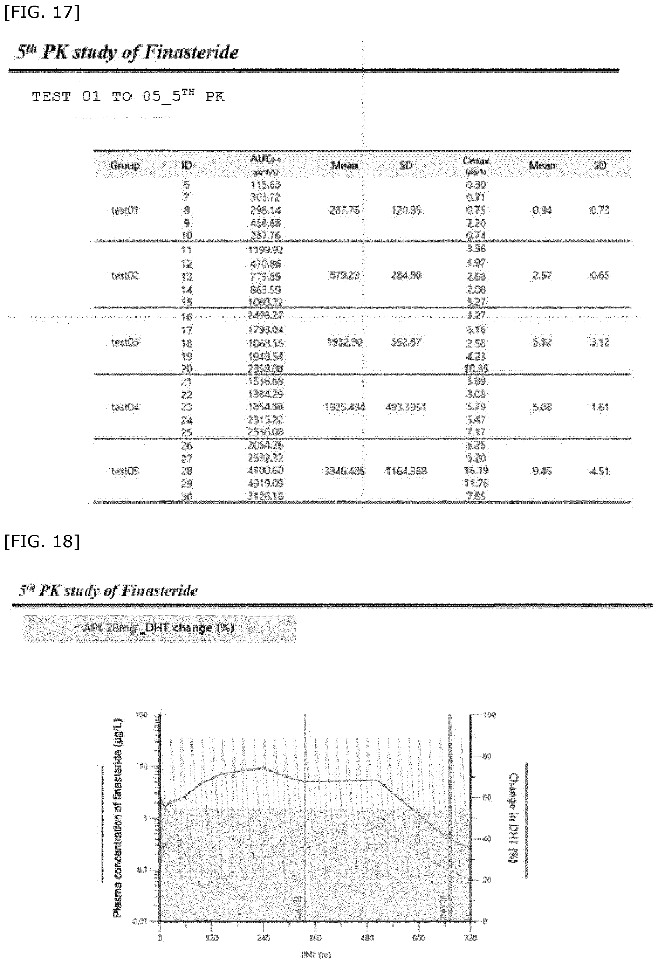

AUC₀₋ₜ is a value calculated by summing the areas under the plasma concentration-time curve calculated from the plasma concentration up to the last measurable blood sampling time point (t) after administration. Cₘₐₓ was calculated as the maximum value among the measured blood concentration values of the respective test subjects. The test results are shown in FIG. 17.

Further, in order to confirm the effect of the released drug, the plasma DHT concentration of a beagle dog was analyzed, and the average blood Finasteride concentration according to time after administration of Injection product 1a (left y-axis, upper line) and the DHT concentration change rate compared to the baseline (right y-axis, lower line) are shown in FIG. 18.

As can be seen from the graph of FIG. 18, it was found that Finasteride in a one-month formulation also did not have a problem of initial excessive release of the drug, and had a sustained drug release effect and an effect of inhibiting DHT production thereby. The DHT production inhibitory effect is to prove the effect of preventing hair loss and treating benign prostatic hyperplasia due to the release of Finasteride.

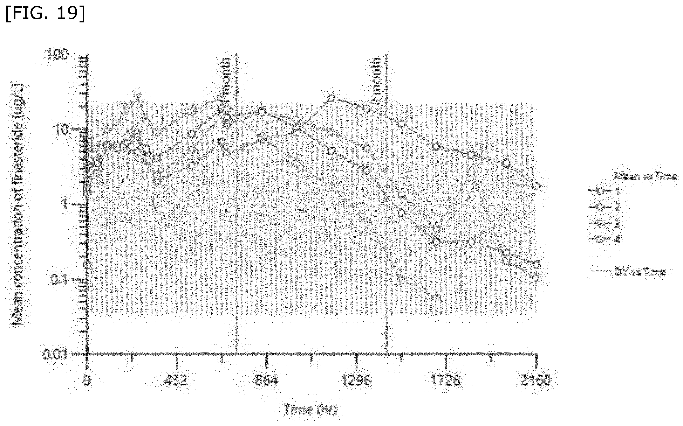

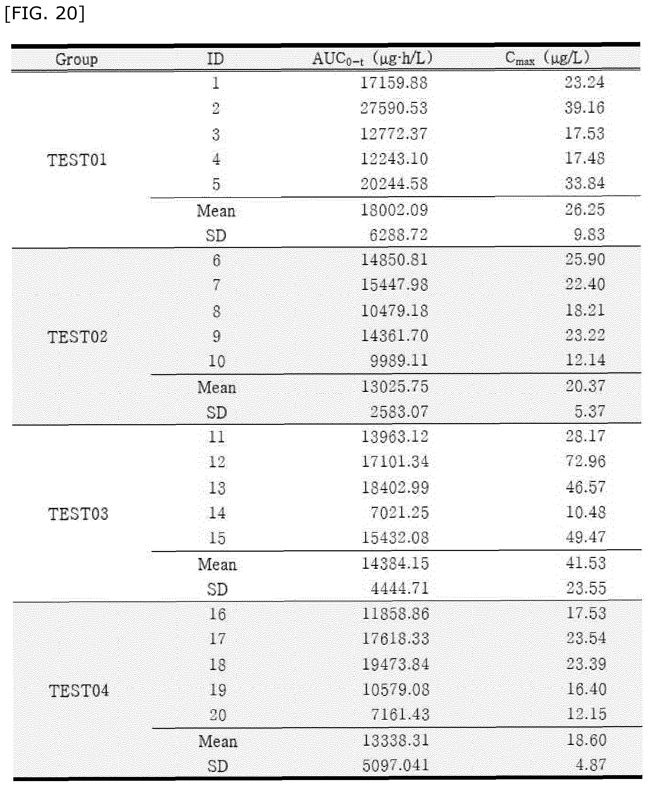

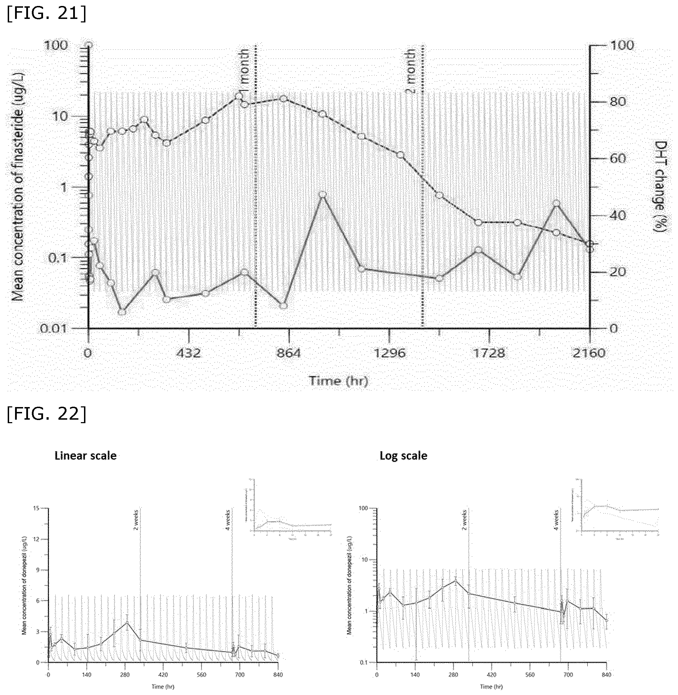

Further, the drug release pattern of Injection product 1b prepared as a three-month Finasteride formulation is shown in FIG. 19. The pharmacokinetic parameters for each administration group of the three-month Finasteride formulation are presented in FIG. 20. Further, the pharmacodynamics of the three-month Finasteride formulation, which is Injection product 1b, are shown in FIG. 21 as average plasma Finasteride concentration according to time after administration (left y-axis, blue line) and plasma DHT concentration change rate (%) obtained based on 100% of the average plasma DHT concentration before drug administration (right y-axis, red line).

As shown in FIG. 21, the three-month Finasteride injection product showed sustained release of the drug for 3 months, and it could be confirmed that the DHT production inhibitory effect appeared for 3 months by this sustained release.

### Test Example 2-2. Donepezil Drug Release

A drug release experiment of the microparticles containing Donepezil of Preparation Example 2 was also performed similarly to Test Example 2-1 above. After a single administration of the composition for injection of Composition Preparation Example 2 above to a beagle dog, the concentration of Donepezil in the blood was quantified using LC-MS/MS. Further, after repeated administration of 10 mg of the oral dosage form Aricept tablet for 3 days, the concentration of Donepezil in the blood was quantified in the same manner as in Preparation Example 2 to compare the blood concentrations and pharmacokinetic parameters with each other. However, in the case of the injection formulation compared to 10 mg of the oral dosage form Aricept tablet (control group, Donepezil hydrochloride), Donepezil was administered in an amount of 63.84 mg, which was 1/4 of a 28-day dosage, as a Donepezil base, and the microparticles were administered in an amount of 351.12 mg.

Pharmacokinetics (PKs) analysis was performed using WinNonlin^{®} software (version 8.0, Pharsight^{®}, Certara^{™} Company).

As a control group, the release pattern of the oral dosage form was simulated and indicated by a black line.

As analysis results of PK parameters for each test subject according to time, area under the plasma concentration-time curve from 0 to the last measurable concentration (AUCₗₐₛₜ) (*µ*g*h/L), mean (Mean), standard deviation (SD), and maximum blood concentration (Cₘₐₓ) (µg/L) were calculated using 'WinNonlin^{®} program (ver. 8.0, Pharsight^{®}, a CertaraTM company)'.

AUCₗₐₛₜ is a value calculated by summing the areas under the plasma concentration-time curve calculated from the plasma concentration up to the last measurable blood sampling time point after administration. Cₘₐₓ was calculated as the maximum value among the measured blood concentration values of the respective test subjects.

The initial blood concentration (Cᵢₙₜ) (*µ*g/L) was calculated through the blood Donepezil concentration data measured after a single administration of the composition for injection of Preparation Example 2 to a beagle dog.

Cᵢₙₜ is the measured initial blood concentration value of each test subject, and is the maximum blood concentration value measured within 24 hours, which is considered an initial excessive release (initial burst effect). Initial burst, as the initial drug release by the drug present on the surface of microparticles, is the initial excessive release concentration that appears before the maximum blood concentration (Cₘₐₓ).

AUCₗₐₛₜ (*µ*g*h/L) and Cₘₐₓ (µg/L) together with the oral dosage form as a control group are as shown in Table 4 and FIG. 22 below.

**[Table 4]**

| Group | ID | AUCₗₐₛₜ(*µ*g*h/L) | | Cₘₐₓ(*µ*g/L) | |
|---|---|---|---|---|---|
| Control group (Oral formulation administration group) | 1 | 118.44 | | 4.59 | |
| | 2 | 81.64 | | 1.83 | |
| | 3 | 158.19 | | 12.59 | |
| | Average | 119.42 | | 6.34 | |
| | SD | 38.28 | | 5.59 | |
| Group | ID | AUCₗₐₛₜ(*µ*g*h/L) | Cᵢₙₜ(*µ*g/L) | Cₘₐₓ(*µ*g/L) | Cint/Cₘₐₓ |
| Test group (Injection product administration group) | 4 | 1551.69 | 1.83 | 4.34 | 0.43 |
| | 5 | 1408.78 | 1.48 | 3.28 | 0.45 |
| | 6 | 1004.10 | 1.13 | 4.74 | 0.24 |
| | Average | 1321.52 | 1.49 | 4.12 | 0.36 |
| | SD | 284.03 | 0.36 | 0.75 | - |

FIG. 22 relates to the release pattern of Donepezil in the blood over time, and the drug release pattern for the injection formulation containing the microparticles of the present disclosure showed a uniform sustained release pattern without showing an initial excessive release.

As can be seen in Table 4 above, the Donepezil injection product showed a Cₘₐₓ close to that of the oral dosage form on the release pattern of the drug. Considering that the dose of the injection product is 1/4 of a one-month dose of the oral formulation, this suggests that a blood concentration sufficient for drug efficacy can be achieved even with a small injection product dose.

Further, when the AUC value (1,114.59 µg*h/L) for 28 days when the oral formulation was repeatedly administered and the AUC value (1,321.52 µg*h/L) of the injection product administration group were compared, the injection product administration group showed a similar rate of 118.57%. This indicates that the AUC value when the injection product according to Preparation Example 2 is administered once is pharmacokinetically equivalent to that when the oral formulation is repeatedly administered for 28 days.

And, when the ratio of the initial blood concentration (Cᵢₙₜ) to the maximum blood concentration (Cₘₐₓ) of the drug was obtained, the average is 0.36, indicating that a constant drug concentration is maintained without initial excessive release.

As such, from the results of the pharmacokinetic experiment showing the drug release pattern of FIG. 22 and the parameters shown in Table 4 above, the Donepezil injection product of the present disclosure, as an injection formulation that provides continuous release for a target period without initial excessive release, can continuously provide the drug efficacy with a dose less than that of the conventional oral dosage form so that it has the effect of not only reducing the inconvenience of taking it every day, but also reducing the occurrence of drug resistance problems.

### Test Example 2-3. Naltrexone Drug Release

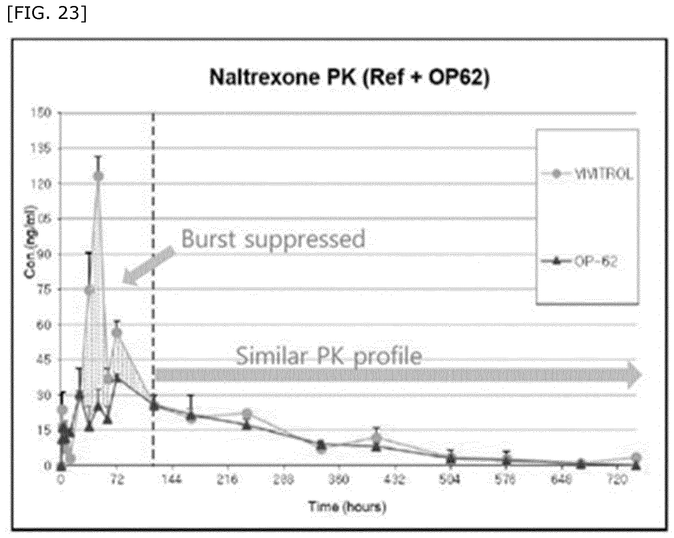

A drug release experiment of the microparticles containing Naltrexone of Preparation Example 3 was also performed similarly to Test Example 2-1 above. The drug release experimental results of the microparticles containing Naltrexone are shown in FIG. 23. FIG. 23 shows a release graph together by performing an experiment together with Vivitrol^{®}, a commercially available injection product of Naltrexone as a comparative group.

As can be confirmed from the graph of FIG. 23, the Naltrexone drug contained in the microparticles of the present disclosure exhibited a remarkably lower drug release pattern up to 72 hours after injection compared to the existing long-acting injection product. This proves that the injection product of the present disclosure does not have a problem of initial excessive release.

Of course, it could be clearly seen that the small average particle diameter and narrow particle size distribution width of the microparticles of the present disclosure play a decisive role in eliminating the initial excessive release problem from the fact that the microparticles of the present disclosure show a release pattern almost similar to the PK profile of the commercial Vivitrol^{®} after about 100 hours after injection.

As described above, the microparticles of the present disclosure do not have an initial excessive release problem compared to microspheres prepared by a conventional process and exhibit a sustained release pattern for a desired period, thereby confirming that a combination of particle properties of particles having a small average particle diameter, a high specific surface area, and a narrow particle distribution width can have a profound effect on the optimized release pattern of sustained-release drugs.

Therefore, since the microparticles of the present disclosure exhibit a sustained release pattern without an initial excessive release, they can be sufficiently prepared as an injection formulation that exhibits controlled release even during various release periods from 1 week to 12 months. Control of such a release period is proved to be able to be achieved through the microparticles of the present disclosure from the fact that the drug release is sustained for several periods such as 1 month, 3 months and 6 months by the particles all of which have a specific surface area per unit mass of 0.75 to 2.0 × 10⁻¹ m²/g while having an average particle diameter of within 20 to 100 um and a particle size distribution width of 35 um or less from the mixing ratio of various drug and biodegradable polymer concentrations as exemplified in Examples above.

In particular, as exemplified through the aforementioned Preparation Examples and Comparative Examples, the microparticles prepared according to the present disclosure may provide injection formulations consisting only of uniform particles that are much smaller in size and have a narrow particle size distribution width compared to the particles prepared by the conventional solvent evaporation method, may enable various drugs to be formed into injection formulations, and may exhibit the lasting effect of the drug as a single injection product without inducing foreign body sensation or pain, thereby having an effect capable of increasing the convenience of administration to patients in need of long-term treatment.

Hereinabove, preferred embodiments of the present disclosure have been exemplified, and the right scope of the present disclosure is not limited by such embodiments, and various variations and modifications to the elements of the present disclosure defined only by the claims below are possible by those skilled in the art, and those skilled in the art will know well that such variations and modifications also belong to the scope of the present disclosure.

### Industrial Applicability

The present disclosure relates to microparticles for sustained release of drugs. More specifically, the present disclosure relates to sustained-release microparticles that contain a drug in microparticles comprising a biodegradable polymer so that there is no excessive release at the initial stage of administration when the drug is administered, and the effective amount of the drug can be continuously released for a certain period of time.

## Claims

1. A sustained-release microparticles comprising a biodegradable polymer and a drug, wherein the biodegradable polymer and the drug are evenly distributed in the microparticles, the microparticles are composed of uniform-sized particles that do not show an initial excessive release of the drug and have a particle size distribution width analyzed by the particle size analyzer of 35 microns or less, and the particles have a specific surface area per unit mass of 0.75×10⁻¹ to 2.0×10⁻¹ m²/g and exhibit a sustained release pattern of the drug for a desired period.

2. The sustained-release microparticles of claim 1, wherein the biodegradable polymer is contained in an amount range of 60 to 97% by weight based on the weight of the microparticles.

3. The sustained-release microparticles of claim 1, wherein the drug is contained in a polymer to drug ratio range of 30:1 to 1.5:1 compared to the biodegradable polymer.

4. The sustained-release microparticles of claim 1, wherein the drug contained in the microparticles is selected from the group consisting of Donepezil, Aripiprazole, Olanzapine, Palonosetron, Minocycline, Memantine, Naltrexone, Alendronate, Deoxycholate, Risedronate, Ibandronate, Zoledronate, Liraglutide, Exenatide, Lanreotide, Octreotide, Deslorelin, Leuprorelin, Goserelin, Triptorelin, and Finasteride.

5. The sustained-release microparticles of claim 1, wherein the biodegradable polymer is one or more selected from the group consisting of poly-L-lactic acid, polylactide, polyglycolic acid, poly-D-lactic acid-co-glycolic acid, poly-L-lactic acid-co-glycolic acid, poly-D,L-lactic acid-co-glycolic acid, polycaprolactone, polyvalerolactone, poly-hydroxybutyrate, and polyhydroxyvalerate.

6. The sustained-release microparticles of claim 1, wherein the microparticles have an average diameter (D₅₀) between 20 um and 100 um and have a smooth spherical surface.

7. The sustained-release microparticles of claim 1, wherein the drug released from the microparticles has a ratio of the initial blood concentration (Cᵢₙₜ) to the maximum blood concentration (Cₘₐₓ) of 1:2 to 1:30.

8. The sustained-release microparticles of claim 1, wherein the desired period includes a period selected from 1 week to 12 months.
